# EUROPEAN PATENT APPLICATION

(11) **EP 2 495 405 A2**
(43) Date of publication of application: **05.09.2012**
(21) Application number: 12170077.7
(22) Date of filing: 06.05.2009
(51) Int. Cl.: F01K 25/14, F01K 21/04

(54) **Power recovery**

(30) Priority: 06.05.2008 GB 0808200; 24.10.2008 US 108233 P
(62) Divisional of application: 09742342.0
(71) Applicant: Invista Technologies S.à.r.l., 9000 St. Gallen (CH)
(72) Inventor: Carrick, Harald, B., Cupar, Fife KY15 7AN (GB); Aird, Graham, Robert, Darlington, Durham DL3 9LU (GB); Humphries, Graeme, Durham, DH1 4RJ (GB)
(74) Representative: Cockerton, Bruce Roger

(57) **Abstract**

The invention relates to a method and apparatus for recovering power from the gaseous stream produced by an oxidation reaction. Specifically, the invention is based on heating the gaseous stream from the oxidation reaction to a temperature of at least 800 DEG C and recovering energy through a gas turbine. The compressor stage of the gas turbine compresses the oxidant feed to the reactor thereby at least partially offsetting the cost of providing the high temperature and pressure reaction conditions in the reactor. The invention also provides improved control of the power recovery system by optimising the efficiency of the gas turbine by feeding gas to the gaseous stream to modulate the flow of gas to the turbine relative to the compressor discharge flow in order to compensate for the consumption of oxidant in the reactor.

## Description

### CROSS-REFERENCE TO RELATED APPLICATIONS

This application claims benefit of priority from Provisional Application No. 61/108233 filed October 24, 2008 and GB Patent Applicatioin No. 0808200.0 filed May 6, 2008.

### FIELD OF THE INVENTION

This invention relates to a method for recovering power from the gaseous stream ("off-gas") produced by an oxidation reaction, for example the oxidations of para-xylene (PX) to terephthalic acid (TA) and dimethyl terephthalate (DMT) or of cyclohexane to cyclohexanone / cyclohexanol. The invention also relates to a reactor comprising a power recovery system. Furthermore, the invention relates to a process for preparing an aromatic carboxylic acid by the liquid phase oxidation of an aromatic carboxylic acid precursor whereby energy is recovered.

### BACKGROUND OF THE INVENTION

Many industrial synthetic chemical processes, for example the synthetic oxidation of para-xylene (PX) to terephthalic acid (TA), and destructive chemical processes, for example the oxidation of organic waste, occur at high temperatures and pressures. The production of TA, for example, typically involves the liquid phase oxidation of PX feedstock using molecular oxygen in acetic acid, in the presence of a dissolved heavy metal catalyst system usually incorporating a promoter, such as bromine as disclosed in US patent number 2,833,816. In general, acetic acid, molecular oxygen in the form of air, para-xylene and catalyst are fed continuously into the oxidation reactor at elevated temperature and pressure, typically a temperature from about 150°C to about 250°C and a pressure from about 600 kPa to about 2500 kPa.

Para-xylene oxidation produces a high-pressure gaseous stream (or "off-gas") which comprises nitrogen, unreacted oxygen, carbon dioxide, carbon monoxide and, where bromine is used as a promoter, methyl bromide. In addition, because the reaction is exothermic, the acetic acid solvent is frequently allowed to vaporize to control the reaction temperature and is removed in the gaseous stream. This vapour is typically condensed and most of the condensate is refluxed to the reactor, with some condensate being withdrawn to control reactor water concentration. The portion of the gaseous stream which is not condensed is either vented, or passed through a catalytic combustion unit (CCU) to form an environmentally acceptable effluent as disclosed in publication WO 96139595. Catalytic combustors have been deployed on TA plants typically upstream of an expander. Their function is to catalytically combust volatile organic compounds (VOC's) and carbon monoxide.

The gaseous stream from the reactor contains a significant amount of energy. This energy can be recovered to offset, at least partially, the cost of obtaining the high temperatures and pressures required in the oxidation reactor. For example, WO 96/11899 and JP 8-155265 disclose directing the high pressure gaseous stream to a means for recovering energy, for example an expander, which is connected to an electric generator or other equipment requiring mechanical work, such as a compressor. Power recovery using an expander (for example as disclosed in WO 96/39595) is conventionally carried out at temperatures from about 150-750 °C, typically 450°C. However, there is scope to improve power recovery using an expander by changes to the configuration of the manufacturing process and the means for recovering power from the process, for example as disclosed in API 616 Gas Turbines for the Petroleum, Chemical and Gas Industry Services.

The TA manufacturing process requires a source of heat above 300 degC to heat the feed stream to the Purification plant hydrogenation reactor. This duty is typically accomplished using a source of High Pressure (HP) Steam (100 bara, 311 degC). Normally, HP steam for this purpose is imported from a Utility provider or raised on site following installation of a packaged boiler assembly.

Similarly, some power for the PTA process is typically provided by a utility provider.

It is therefore an object of the invention to provide an improved power recovery system with potential for reduced plant HP steam usage and method for recovering more power along with generating HP steam from the gaseous streams of oxidation reactions.

### SUMMARY OF THE INVENTION

In accordance with the present invention, a method has been found to improve power recovery from an oxidation reactor by heating the gaseous stream from the oxidation reaction to higher temperatures (for example at least 800°C) and recovering energy through an expander (turbine). At such temperatures expanders provide significantly improved power recovery relative to expanders at about 450°C, the improved power recovery more than offsets the additional cost of heating the off-gas. The additional power recovered from the higher temperature gaseous stream can be utilised elsewhere in the oxidation process. The present invention can be characterized by a method for recovering power from an oxidation reaction producing a gaseous stream, the reaction being conducted in a continuous oxidation reactor fed with gaseous oxidant, comprising:
(a) heating the gaseous stream to a temperature of at least 800°C;
(b) feeding the gaseous stream to a gas turbine comprising a turbine coupled to a compressor, where the compressor compresses the gaseous oxidant fed to the reactor.

### BRIEF DESCRIPTION OF DRAWINGS

The figures describe examples of the different embodiments (configurations and modes) of the present invention.

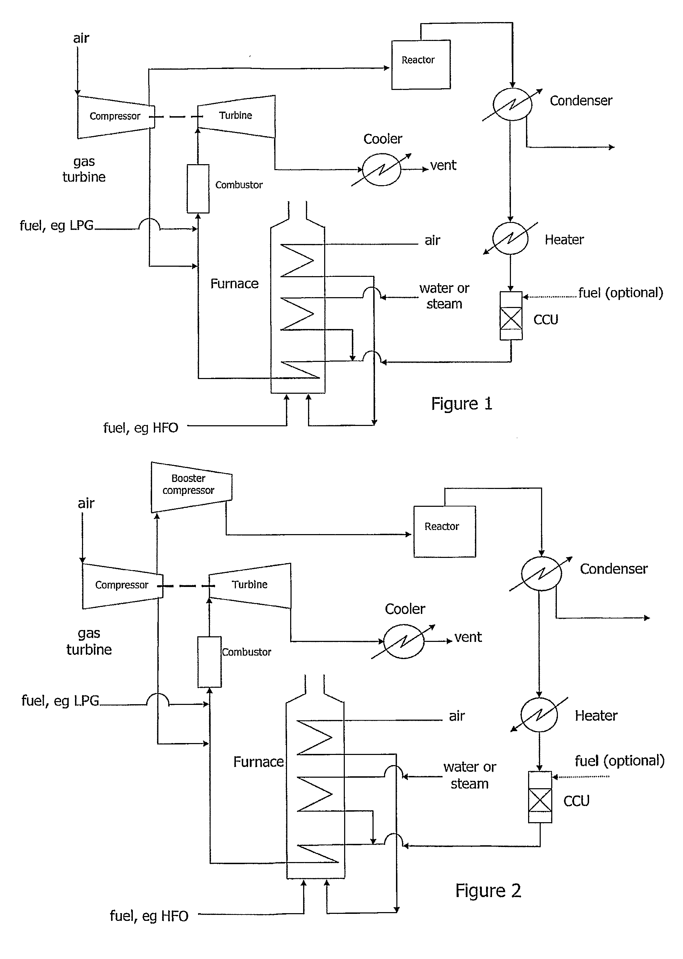

Figure 1 is a schematic process diagram which illustrates one embodiment of the invention in which: Air is compressed in the compressor of the gas turbine and fed to an oxidation reactor in which it is reacted. The gaseous stream from the reactor is condensed to remove condensibles, heated and then reacted in a catalytic combustion unit. Steam or gas is heated in the indirect heater and then added to the catalytically combusted gaseous stream prior to it being heated in an indirect heater. Fuel and air preheated in the indirect heater are fed to the heater to provide a heat source. The resulting hot gaseous stream is then contacted with a clean fuel and with make-up air from the compressor discharge and reacted in a direct-fired combustor to generate the desired gas temperature. The combustor off-gas is then fed to the turbine from which power is recovered. The hot vent gases from the turbine are cooled to recover heat and then discharged to atmosphere.
Figure 2 is a schematic diagram of another alternative embodiment of the invention in which: Air is compressed in the compressor of the gas turbine then further compressed in a booster compressor. The compressed air is fed to an oxidation reactor in which it is reacted. The gaseous stream from the reactor is condensed to remove condensibles, heated and then reacted in a catalytic combustion unit. Steam or gas is heated in the indirect heater and then added to the catalytically combusted gaseous steam prior to its being heated in an indirect heater. Fuel and air are preheated in the indirect heater then fed to the heater to provide a heat source. The resulting hot gaseous stream is then contacted with a clean fuel and with make-up air from the compressor discharge and reacted in a direct-fired combustor to generate the desired gas temperature. The combustor off-gas is then fed to the turbine from which power is recovered. The hot vent gases from the turbine are cooled to recover heat and then discharged to atmosphere.

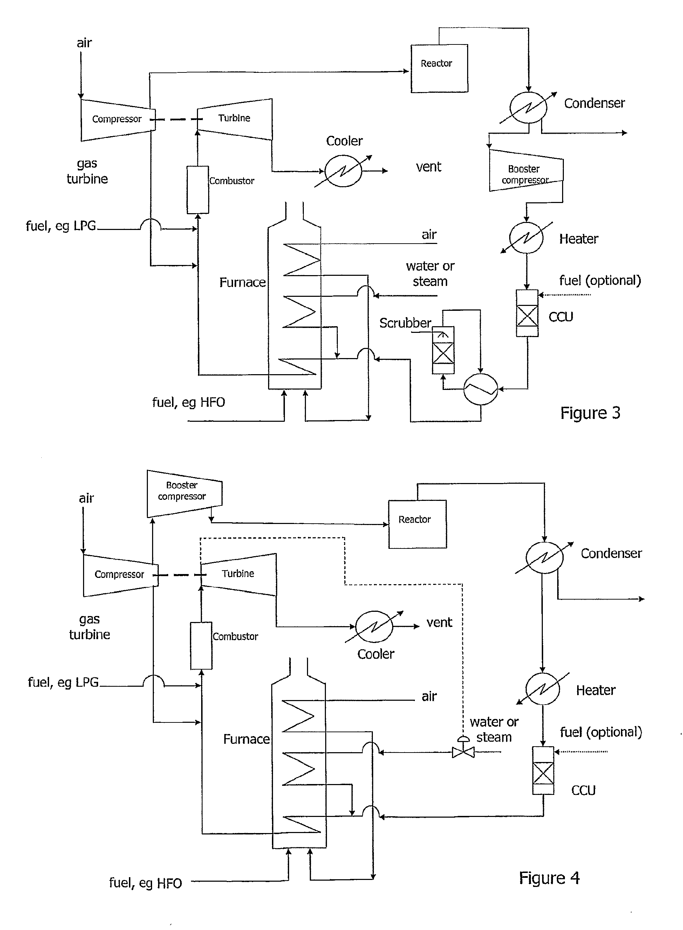

Figure 3 is a schematic diagram of another alternative embodiment of the invention in which: Air is compressed in the compressor of the gas turbine and fed to an oxidation reactor in which it is reacted. The gaseous stream from the reactor is condensed to remove condensibles, heated then reacted in a catalytic combustion unit. The gaseous stream is cooled and treated, for example by a scrubber to remove reactive components such as HBr and Br₂ and then compressed in a booster compressor. The booster compressor is used to maintain the required pressure of the gaseous stream from the reactor through gas treatment to the turbine. Steam or gas is heated in the indirect heater and added to the treated and reheated gaseous stream prior to it being heated in an indirect heater. The resulting hot gaseous stream is then contacted with a clean fuel and with make-up air from the compressor discharge and reacted in a direct-fired combustor to generate the desired gas temperature. The combustor off-gas is then fed to the turbine from which power is recovered. The hot vent gases from the turbine are cooled to recover heat and then discharged to atmosphere.
Figure 4 is a schematic diagram of another alternative embodiment of the invention in which: Air is compressed in the compressor of the gas turbine and further compressed in a booster compressor. The compressed air is fed to an oxidation reactor in which it is reacted. The gaseous stream from the reactor is condensed to remove condensibles, heated and then reacted in a catalytic combustion unit. Steam or gas, fed under flow or pressure control from the feed to the turbine, is heated in the indirect heater and then added to the catalytically combusted gaseous stream prior to being heated in an indirect heater. Fuel and air are preheated in the indirect heater then fed to the heater to provide a heat source. The resulting hot gaseous stream is then contacted with a clean fuel and with make-up air from the compressor discharge and reacted in a direct-fired combustor to generate the desired gas temperature. The combustor off-gas is then fed to the turbine from which power is recovered. The hot vent gases from the turbine are further cooled to recover heat and then discharged to atmosphere.

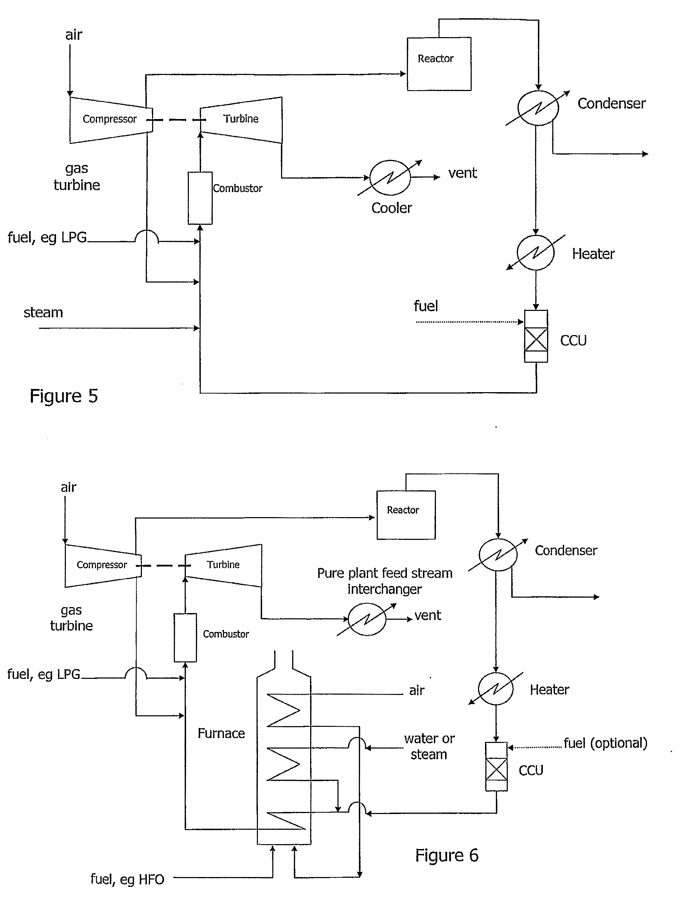

Figure 5 is a schematic diagram of another alternative embodiment of the invention in which: Air is compressed in the compressor of the gas turbine, then fed to an oxidation reactor in which it is reacted. The gaseous stream from the reactor is condensed to remove condensibles, heated and reacted in a catalytic combustion unit. Steam or gas is added to the gaseous stream, contacted with a clean fuel and with make-up air from the compressor discharge and reacted in a direct-fired combustor to generate the desired gas temperature. The combustor off-gas is then fed to the turbine from which power is recovered. The hot vent is further cooled to recover heat and then discharged to atmosphere.
Figure 6 is a schematic diagram of another alternative embodiment of the invention in which: Figure 1 is modified by interchanging the hot vent gases from the turbine with the feed to the purification plant to displace the high pressure steam requirement from that duty.

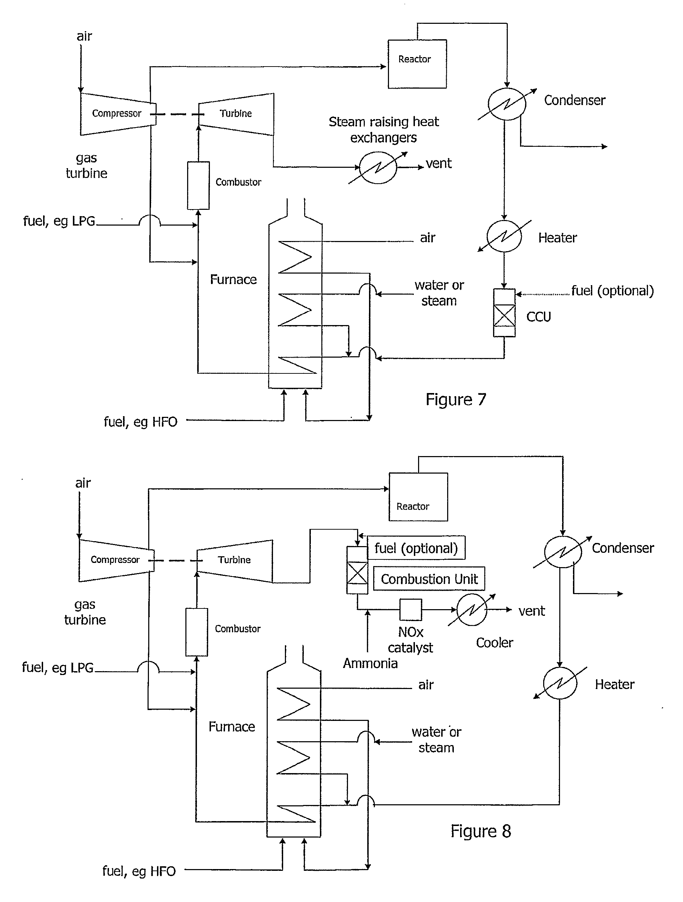

Figure 7 is a schematic diagram of another alternative embodiment of the invention in which: Figure 1 is modified by using the hot vent gases from the turbine to raise steam, which can be high temperature steam (for example 300- 500 degC)
Figure 8 is a schematic diagram of another alternative embodiment of the invention in which: Figure 1 is modified by adding a combustion unit downstream of the LP Turbine. Additionally ammonia injection together with a NOx reduction catalyst is shown. The catalytic combustion unit in Figure 1 that was downstream of the "Heater" has been removed.

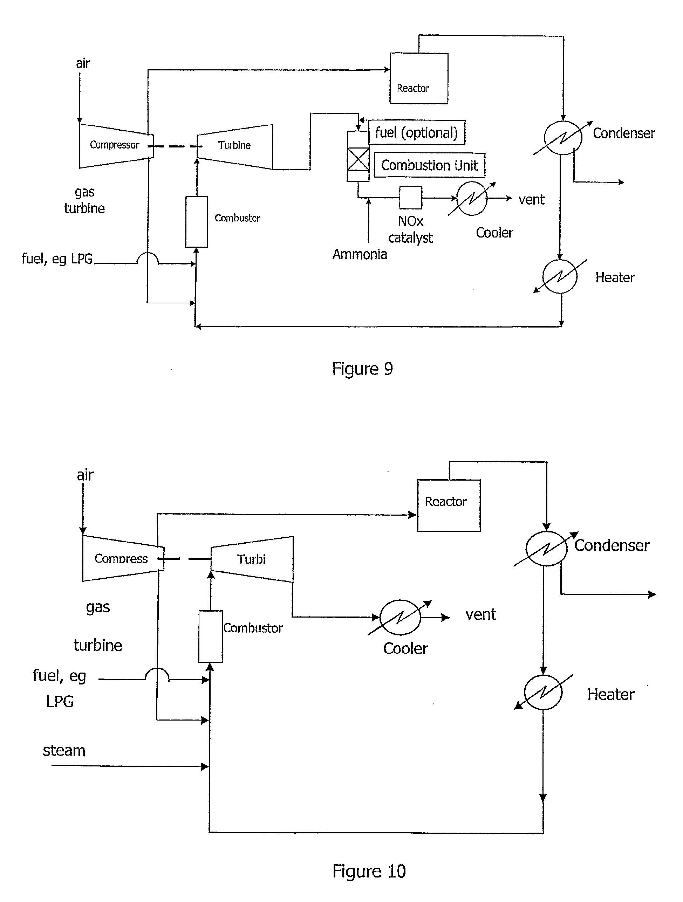

Figure 9 is a schematic diagram of another alternative embodiment of the invention in which: Figure 8 is depicted with the furnace removed.
Figure 10 is a schematic diagram of another alternative embodiment of the invention in which: Figure 5 is modified by removing the CCU unit.

In any of these configurations a generator can be attached to the turbine. The net power generated can be used to offset the power requirement of the PTA plant. Surplus power can be exported from the plant.

### DETAILED DESCRIPTION OF TFIE INVENTION

The present invention can be characterized by a method for recovering power from an oxidation reaction producing a gaseous stream, the reaction being conducted in a continuous oxidation reactor fed with gaseous oxidant, comprising:
(a) heating the gaseous stream to a temperature of at least 800°C;
(b) feeding the gaseous stream to a gas turbine comprising a turbine coupled to a compressor, where the compressor compresses the gaseous oxidant fed to the reactor.

Gas can be added to the gaseous stream prior to, or simultaneously with, feeding the gaseous stream to the turbine. The mass flow of the gas added to the gaseous stream can be in the range of from about 0% to about 25% of the mass flow of the gaseous stream prior to addition of the gas, for example from about 6% to about 15%. The gas added to the gaseous stream can be steam or air. The gaseous stream can be heated to a temperature in the range of from 800°C to about 1300°C, for example 800°C to about 1100°C, or suitably about 1050°C. An expander or booster compressor can be provided downstream of the gas turbine compressor on the gaseous oxidant inlet to the oxidation reactor. A heater can be provided to heat the gaseous stream and a booster compressor can be provided downstream of the oxidation reactor and upstream of the heater. The gaseous stream can be heated with a direct heater or an indirect heater. The heater can be an interchanger. The gaseous stream can be heated with a catalytic combustion unit prior to heating with a direct heater, an indirect heater or an interchanger. The gaseous stream can be treated with a scrubber. The process can further comprise generating steam from the gaseous stream after step (b). The process can further comprise generating electricity from the gaseous stream after step (b). The can further comprise removing CO and NOx from the gaseous stream after step (b).

Another embodiment of the present invention can be characterized by a method of monitoring power recovery from an oxidation reaction producing a gaseous stream, the reaction being conducted in a continuous oxidation reactor fed with gaseous oxidant, comprising:
(a) heating the gaseous stream to a temperature of at least 800°C;
(b) feeding the gaseous stream to the turbine stage of a gas ICOCGT comprising a turbine coupled to a compressor, where the compressor compresses the gaseous oxidant fed to the reactor;
(c) monitoring the pressure at the turbine stage of the ICOCGT;
(d) maintaining the pressure within the turbine stage of the ICOCGT within a pressure range above a minimum value corresponding to the power demand of the compressor to compress the gaseous oxidant feed to an oxidation reactor and below a maximum value set by the power or pressure limits of the gas turbine by adding gas to the gaseous stream.

The flow of the added gas can be varied to control the pressure at the turbine stage of the gas turbine and the power output of the turbine stage. Gas can be added to the gaseous stream prior to, or simultaneously with, feeding the gaseous stream to the turbine. The mass flow of the gas added to the gaseous stream can be in the range of from about 0% to about 25% of the mass flow of the gaseous stream prior to addition of the gas, for example from about 6% to about 15%. The gas added to the gaseous stream can be steam or air. The gaseous stream can be heated to a temperature in the range of from 800°C to about 1300°C, for example 800°C to about 1100°C, or suitably about 1050°C. An expander or booster compressor can be provided downstream of the gas turbine compressor on the gaseous oxidant inlet to the oxidation reactor. A heater can be provided to heat the gaseous stream and a booster compressor can be provided downstream of the oxidation reactor and upstream of the heater. The gaseous stream can be heated with a direct heater or an indirect heater. The heater can be an interchanger. The gaseous stream can be heated with a catalytic combustion unit prior to heating with a direct heater, an indirect heater or an interchanger. The gaseous stream can be treated with a scrubber. The process can further comprise generating steam from the gaseous stream after step (d). The process can further comprise generating electricity from the gaseous stream after step (d). The can further comprise removing CO and NOx from the gaseous stream after step (d).

Another embodiment of the present invention can be characterized by a continuous reactor for an oxidation reaction producing a gaseous stream, the reactor comprising:
(a) a vessel comprising an oxidant inlet and a gaseous stream outlet; and
(b) a power recovery system connected to the gaseous stream outlet comprising:
   (b1) a heater for heating the gaseous stream connected downstream of the gaseous stream outlet; and
   (b2) a gas turbine connected downstream of the heater comprising a turbine coupled to a compressor, where the compressor is connected to the oxidant inlet such that, when the reactor is in use, the compressor compresses the gaseous oxidant fed to the reactor.

Prior to, or simultaneously with, feeding the gaseous stream to the turbine, a gas (for example steam or air) can be added to the gaseous stream. In the reactor of the invention, therefore, the power recovery system can comprise a gas inlet for adding gas to the gaseous stream. The heater can be a direct heater or an indirect heater. A catalytic combustion unit can be provided upstream of the heater. A scrubber can be provided between the catalytic combustion unit and the heater. The power recovery system can further comprise an expander or booster compressor downstream of the gas turbine compressor on the gaseous oxidant inlet to the oxidation reactor. The power recovery system can further comprise a booster compressor downstream of the oxidation reactor and upstream of the heater. The power recovery system can further comprise generating steam from the gaseous stream after step (b2). The power recovery system can further comprise generating electricity from the gaseous stream after step (b2). The power recovery system can further comprise removing CO and NOx from the gaseous stream after step (b2).

Another embodiment of the present invention can be characterized by a process of oxidizing a precursor to an aromatic carboxylic acid or ester thereof in a liquid phase reaction mixture, whereby energy is recovered from the oxidation reaction, comprising:
(a) contacting one or more precursors of the aromatic carboxylic acid with an oxidant, in the presence of a catalyst and a liquid phase solvent, in a continuous oxidation reactor fed with gaseous oxidant to produce aromatic carboxylic acid and a gaseous stream;
(b) heating the gaseous stream to a temperature of at least 800°C;
(c) feeding the gaseous stream to a gas turbine comprising a turbine coupled to a compressor, where the compressor compresses the gaseous oxidant fed to the reactor.

Gas can be added to the gaseous stream prior to, or simultaneously with, feeding the gaseous stream to the turbine. The mass flow of the gas added to the gaseous stream can be in the range of from about 0% to about 25% of the mass flow of the gaseous stream prior to addition of the gas, for example from about 6% to about 15%. The gas added to the gaseous stream can be steam or air. The gaseous stream can be heated to a temperature in the range of from 800°C to about 1300°C, for example 800°C to about 1100°C, or suitably about 1050°C. An expander or booster compressor can be provided downstream of the gas turbine compressor on the gaseous oxidant inlet to the oxidation reactor. A heater can be provided to heat the gaseous stream and a booster compressor can be provided downstream of the oxidation reactor and upstream of the heater. The gaseous stream can be heated with a direct heater or an indirect heater. The heater can be an interchanger. The gaseous stream can be heated with a catalytic combustion unit prior to heating with a direct heater, an indirect heater or an interchanger. The gaseous stream can be treated with a scrubber. The process can further comprise generating steam from the gaseous stream after step (c). The process can further comprise generating electricity from the gaseous stream after step (c). The process can further comprise removing CO and NOx from the gaseous stream after step (c).

Another embodiment of the present invention can be characterized by a method for raising power from fuel using the gas turbine. This power generated can be used to offset the plant power requirement. However, more power can be raised than is required for the PTA plant itself. Surplus power generated from the gas turbine can be exported to another chemical plant or to a utility company.

Another embodiment of the present invention can be characterized by a method for heating feeds to the Purification plant hydrogenation reactor, for example this can be accomplished by either:
(a) direct heat exchange, namely using the hot turbine exhaust gas to directly heat the Purification plant hydrogenation reactor feed stream;
(b) using the hot turbine exhaust gas to raise HP steam. The HP steam is then used to heat the Purification plant hydrogenation reactor feed stream.

An Internal Combustion Open Cycle Gas Turbine (ICOCGT), as disclosed in API 616 Gas Turbines for the Petroleum, Chemical and Gas Industry Services, comprises a compressor, a combustor and a turbine and is optimized to generate power. An embodiment of the present invention utilizes an ICOCGT to beneficially recover power from the gaseous stream produced by an oxidation reaction.

The compressor stage of the ICOCGT compresses the oxidant feed to the reactor (at greater than atmospheric pressure) thereby at least partially offsetting the cost of providing the high temperature and pressure reaction conditions in the reactor.

The turbine stage of the ICOCGT expands the heated gaseous stream from the oxidation reactor recovering energy to power the compressor and a hot gas stream, for example to raise steam downstream of the ICOCGT. The net power generated can be used to offset the power requirement of the PTA plant. Surplus power can be exported from the plant.

Another embodiment of the invention can be characterized by improved control of the power recovery system. The inventors have discovered that the performance of the ICOCGT can be improved by feeding gas (for example steam or air) to the gaseous stream to modulate the flow of gas to the turbine relative to the compressor discharge flow in order to compensate for the consumption of oxidant in the reactor. This embodiment of the present invention therefore provides a convenient method for optimising the power recovery by monitoring the pressure at the turbine stage of the gas turbine. Steam can be utilised in this method because steam is readily available and is usually a by-product of the reaction process.

In another embodiment of the present invention an expander or booster compressor can be provided downstream of the gas turbine compressor on the oxidant inlet to the oxidation reactor or downstream of the oxidation reactor to adjust the gas turbine compressor discharge to match the optimum pressure of gaseous oxidant into the reactor. Together, the modulation of steam flow into the gaseous stream and the expander or booster compressor enables the use of an ICOCGT coupled to a chemical reactor, thereby allowing the reactor and gas turbine operations to be optimised independently. This embodiment therefore allows a standard gas turbine, which may not itself have optimum properties for the power recovery system, to be optimally integrated for efficient power recovery.

These, and other aspects of the invention, are described in detail below.

### Heating of the gaseous stream

Prior to heating the gaseous stream or mixing the gaseous stream with steam, solvent in the gaseous steam (for example acetic acid in TA production) can be condensed, for example using separation apparatus such as a distillation column or overhead condensers. Usually, most of the condensate is refluxed to the reactor, with some of the condensate being withdrawn to control reactor water concentration (two moles of water are formed per mole of PX reacted in TA production).

When it leaves the reactor, the gaseous stream typically has a temperature from 150 to 220°C and a pressure from 600 kPa to 2500 kPa. The temperature and pressure of the reactor can be selected to optimize the operation of the reactor and the downstream processes.

Optionally, to optimize the temperature and pressure of the gaseous stream leaving the reactor it can be necessary to boost the gaseous stream pressure in an additional compressor.

The gaseous stream leaving the reactor can be heated to at least 800°C with any suitable heater, such as a direct heater of the gaseous stream, such as a combustor fuelled for example with natural gas or fuel oil, or an indirect heater of the gaseous stream, such as a furnace fuelled for example with natural gas or fuel oil. In one embodiment, fuel and oxidant (for example from the reactor oxidant feed) are mixed with the gaseous stream in a combustor and burnt in order to raise the temperature of the gaseous stream directly. Typically, however, a furnace heats the gaseous stream indirectly, i.e. fuel and oxidant (for example air) are burnt in the furnace without mixing with the gaseous stream and the gaseous stream is heated by indirect heat exchange as it passes thorough the furnace. Indirect heating can be advantageous as it does not require additional oxidant to be fed above atmospheric pressure to the gaseous stream to burn the fuel. Instead indirect heating can involve combustion of fuel using atmospheric air.

Optionally, other auxiliary heaters can be used in addition to the heater for heating the gaseous stream. Prior to heating (i.e. upstream of the heater), the gaseous stream can be fed to a catalytic combustion unit (CCU). CCUs are typically used for environmental reasons to remove organic compounds and reactor by-products in the gaseous stream and operate by flameless oxidation of the organic compounds etc (e.g. MeBr). Typically, the gaseous stream leaving the CCU has a temperature of from about 450°C to about 600°C. Optionally, the gaseous stream can be heated with an interchanger, i.e. a heat exchanger that transfers heat between a process stream and the gaseous stream.

The temperature of the gaseous stream entering the CCU can be about 250°C to about 400°C, for example about 300°C, to ensure stable combustion in the CCU. Prior to treatment with the CCU, the gaseous stream can be heated from about 200°C to about 350°C, for example from about 300°C to about 350°C. A steam heater provided upstream of the CCU can be used to achieve such heating. The steam heater can use steam produced as a by-product of the oxidation reaction to heat the gaseous stream.

Following the CCU, optionally the gas can be treated, for example by scrubbing (for example by use of a scrubber), to remove reactive components such as HBr and Br₂ prior to feeding to a gas heater. One way of heating the gaseous feed stream to the CCU can be to interchange heat with the CCU exit stream.

### Addition of gas to the gaseous stream

Prior to, or simultaneously with, feeding the gaseous stream to the turbine inlet, gas (for example steam or air) can be added to the gaseous stream. In the reactor of the invention, therefore, the power recovery system can comprise a gas inlet for adding gas to the gaseous stream. The gas, for example steam or air, can be added to the gaseous stream before or after the step of heating the gaseous stream (or where heating involves more than one step between steps). The gas can be added before the step of heating of the gaseous stream (i.e. upstream of the heater). The gaseous stream, immediately prior to being fed to the turbine, has a temperature of at least 800°C, for example in the range of from 800°C to about 1300°C, or 800°C to about 1100°C, or suitably about 1050°C.

The gas can be added to the gaseous stream to modulate the mass flow of gas to the turbine to compensate for the oxidant consumed in the oxidation reactor. To optimise the economic performance additional gas can be further added to the gaseous stream beyond that required to compensate for the consumption of oxidant in the reactor. This can be advantageous, as it allows the power recovered in the gas turbine to be increased. Calculation of the gas turbine characteristics can allow determination of the additional gas added to the gaseous stream; the upper value set by the power or pressure limits of the gas turbine and the lower value set by generating sufficient power to drive the compressor. Typically, however, the mass flow of gas added to the gaseous stream can be in the range of from about 0% to about 25%, for example about 6% to about 15%, of the mass flow of the gaseous stream (prior to addition of gas).

Alternatively, the additional flow of gas can allow the temperature of gaseous stream flowing into the turbine to be lowered (while achieving equivalent power recovery from the gas turbine), thus reducing the temperature to which the gaseous stream must be heated and saving fuel costs. In general, the power recovery by the gas turbine can be optimised by the selection of the appropriate gaseous stream flow into the gas turbine (which can be modulated by the addition of steam or air) and the temperature of the gaseous stream into the turbine.

### The gas tur-bine

As used herein, a "gas turbine" refers to a standard gas turbine, for example those described and listed in API 616 Gas Turbines for the Petroleum, Chemical and Gas Industry Services and Turbomachinery International Handbook 2006, vol. 46, no. 6, comprising a compressor coupled to a turbine by one or more shafts. The turbine is connected to the gaseous stream downstream of the heater. The compressor is connected to the oxidant inlet of the reactor and compresses the gaseous oxidant fed to the reactor. Typically, the turbine power will be greater than the compressor power consumption.

Because compressing the oxidant (which is used in significant quantities in the reactor) is a costly step in the reaction process, it is advantageous that this cost be at least partially offset by power recovery from the gaseous stream.

To minimise the capital cost of the power recovery system, the gas turbine used in the invention can be of a standard design and construction with only minor modification. Generally, the present invention selects a gas turbine designed for the temperatures, pressures and flow rates of the gaseous stream, and the power requirements of the compressor for compressing the oxidant feed. An expander or booster compressor can be provided downstream of the gas turbine compressor on the gaseous oxidant feed. This expander or compressor allows adjustment of the turbine compressor discharge to match the optimum pressure of gaseous oxidant into the reactor in order to assist with the integration of the gas turbine with the remaining components of the power recovery system and the reactor, and to allow optimisation of the power recovery. This embodiment can be particularly advantageous, as it enables de-coupling of the requirements of the gas turbine and reactor, thereby allowing the reactor and gas turbine operations to be optimised independently. Alternatively, a booster compressor can be located downstream of the oxidation reactor and upstream of the heater to adjust and optimise the pressure of the gaseous stream into the turbine.

Gas (for example steam or air) can be added to the gaseous stream, prior to, or simultaneously with, feeding the gaseous stream to the turbine inlet. In one embodiment, gas can be added to the gaseous stream prior to feeding the gaseous stream to the turbine inlet (i.e. upstream of the turbine) and, therefore, in the power recovery system of the invention, the steam inlet is upstream of the turbine. This can be advantageous to match the compressor and turbine duties, enabling the use of a standard gas turbine.

### Downstream of the gas turbine

One technical issue of the present invention is ensuring that the off gas emissions meet increasingly tight NOx and CO legislative specifications. With respect to the turbine burner design, in general, higher combustion temperature and longer residence time encourages good CO combustion at the expense of increased production of NOx. The converse also applies - low combustion temperature or, residence time leads to low NOx but also to high CO. Where unacceptably high CO or NOx is left in the offgas the gaseous stream would need to be treated. Finding a suitable design point gives significant risk of the operating plant failing to meet at least one of its environmental operating targets either through inaccuracy of the design to exacting requirements or due to need of the PTA plant for operational flexibility.

To mitigate this risk the gaseous stream downstream of the turbine can be treated as follows:
1. The gaseous stream is fed to a combustion unit (catalytic or non-catalytic) to remove CO as conventionally known in the art.
2. Following the combustion unit, ammonia can be injected to the gas stream and directed over a catalyst bed to reduce the NOx to N2 gas. This stream can then be discharged to atmosphere meeting relevant enviromnental standards.

Either, or both, of these schemes may be used depending upon the local environmental standards required.

### The reactor, oxidation reactants and conditions

The reactor is a continuous flow reactor, meaning a reactor in which reactants are introduced and mixed and products withdrawn simultaneously in a continuous manner, as opposed to a batch-type reactor. In this invention a standard oxidation reactor, for example as disclosed in US 7,153,480, can be used. Standard reactants and operating conditions, for example as disclosed in US 7,153,480, can also be used.

The invention is suitable for any oxidation reaction producing a gaseous stream, i.e. gaseous reaction products. For example, oxidation reactions include cyclohexane oxidation, PX oxidation to TA or dimethyl terephthalate, metaxylene oxidation to isophthalic acid etc. However, the oxidation of PX to TA is of particular interest in the invention.

The oxidant in the invention can be molecular oxygen, for example air (including oxygen-depleted air and oxygen enriched air).

Oxidation reactions are typically exothermic and heat can be removed, in order to control the reaction temperature, by removing the volatile components, condensing them, and returning the condensate to the reactor. Alternatively or additionally, the heat of reaction can be removed from the reaction by heat exchange with a heat-accepting fluid, according to conventional techniques known to those skilled in the art.

As mentioned above, the reactor is generally operated in a continuous mode. By carrying out the process in a continuous flow reactor, the residence time for the reaction can be made compatible with the attainment of conversion of the precursors to the desired product without significant production of degradation products.

The gaseous stream can be heated to a temperature in the range of from 800°C to about 1300°C, for example 800°C to about 1100°C, or suitably about 1050°C.

As used herein, reference to the production of a carboxylic acid includes reference to the production of its ester. As will be evident to the skilled person, whether a carboxylic acid or its ester is produced will depend on the conditions in the reactor and/or the conditions used to purify the products.

As used herein, "aromatic carboxylic acid precursor" or "precursor" means an organic compound, preferably a hydrocarbon, capable of being oxidised to a specific aromatic carboxylic acid in a majority yield in the presence of selective oxidising conditions. An example of a terephthalic acid precursor is paraxylene. An example of an isophthalic acid precursor is metaxylene.

The present invention can comprise feeding solvent, oxidant, precursor and catalyst into an oxidation reactor that is maintained at a temperature in the range of from about 150°C to about 250°C, for example about 175°C to about 225°C, and a pressure in the range of from about 100 kPa to about 5000 kPa, for example about 1000 kPa to about 3000 kPa.

The oxidation reaction can be carried out in the presence of an oxidation catalyst. The catalyst can be substantially soluble in the reaction medium comprising solvent and the aromatic carboxylic acid precursor(s). The catalyst can comprise one or more heavy metal compounds, for example cobalt and/or manganese compounds, and can optionally include an oxidation promoter. For instance, the catalyst can take any of the forms that have been used in the liquid phase oxidation of aromatic carboxylic acid precursors such as terephthalic acid precursor(s) in aliphatic aromatic carboxylic acid solvent, for example bromides, bromoalkanoates or alkanoates (usually C₁-C₄ alkanoates such as acetates) of cobalt and/or manganese. Compounds of other heavy metals such as vanadium, chromium, iron, molybdenum, a lanthanide such as cerium, zirconium, hafnium, and/or nickel can be used instead of, or additional to, cobalt and/or manganese. Advantageously, the catalyst system can include manganese bromide (MnBr₂) and/or cobalt bromide (CoBr₂). The oxidation promoter, where employed, can be in the form of elemental bromine, ionic bromide (for example HBr, NaBr, KBr, NH4Br) and/or organic bromide (for example bromobenzenes, benzyl-bromide, mono- and di-bromoacetic acid, bromoacetyl bromide, tetrabromoethane, ethylene-dibromide, etc.).

Any suitable solvent in which the oxidation reaction can take place can be used. Where the oxidation reaction is the catalytic liquid phase oxidation of a precursor to produce aromatic carboxylic acid, the solvent can be an aliphatic monocarboxylic acid having from 2 to 6 carbon atoms, for example, the solvent can be acetic acid. Acetic acid can be particularly useful as the solvent since it is relatively resistant to oxidation in comparison with other solvents and increases the activity of the catalytic pathway.

The reaction can be effected by heating and pressurising the precursor, catalyst and solvent mixture followed by introduction of the oxidant into the reactor via the oxidant inlet.

The effluent, i.e. reaction product, from the oxidation reactor can be a slurry of aromatic carboxylic acid crystals which are recovered from the slurry by filtration and subsequent washing. They can thereafter be fed to a separate purification step or directly to a polymerization process, for example, the main impurity in crude TPA is 4-carboxybenzaldehyde (4-CBA), which is incompletely oxidized paraxylene, although other oxidation products and precursors to terephthalic acid such as p-tolualdehyde and p-toluic acid can also be present as contaminants.

### Start-up and shut-down

A method of starting the power recovery system can be to start the gas turbine using its normal starting device (such as a starter motor), obeying all the normal protocols for internal combustion gas turbine operation. This stage of start up uses a plant bypass shortcutting the whole reactor system and heating train (comprising furnace etc.). Once the gas turbine is in steady state at part-load, air is gradually passed through the booster compressor or expander (if present), and heating train (comprising furnace etc.) and returned to the gas turbine combustor and the expansion stage of the gas turbine. Similarly, if a booster compressor is located upstream of the combustor, this is started once the gas turbine is in steady state at part load. This stage of start up uses an air bypass round the oxidation reactor. After all the air from the compressor of the gas turbine is being fed through the heating train the first bypass is fully closed. Now air is fed to the oxidation reactor in order to initiate the reaction. Steam is fed to the heating train to maintain the gas turbine output power to the desired level. The proportion of air fed to the reactor is controlled by modulating the reactor bypass valve. The power recovery system is fully online once the reactor bypass is closed.

On oxidation reactor trip, the air flow from the compressor stage is diverted through the reactor bypass to the expansion stage of the gas turbine. The reactor can be restarted readily from this condition. Shutdown of the gas turbine can also proceed from this condition.

### General

The term "comprising" encompasses "including" as well as "consisting" for example a composition "comprising" X may consist exclusively of X or may include something additional for example X + Y.

The term "about" in relation to a numerical value x means, for example, x±10%.

It will be understood that the invention has been described by way of example only and modifications can be made whilst remaining within the scope and spirit of the invention.

The invention also encompasses the following embodiments.
1. A method for recovering power from an oxidation reaction producing a gaseous stream, the reaction being conducted in a continuous oxidation reactor fed with gaseous oxidant, comprising:
   (a) heating the gaseous stream to a temperature of at least 800°C;
   (b) feeding the gaseous stream to a turbine stage of a gas turbine comprising a turbine coupled to a compressor, where the compressor compresses the gaseous oxidant fed to the reactor.
2. The method of embodiment 1 wherein prior to, or simultaneously with, feeding the gaseous stream to the turbine, gas is added to the gaseous stream.
3. The method of embodiment 1 or claim 2 wherein an expander or booster compressor is provided downstream of the gas turbine compressor on the gaseous oxidant inlet to the oxidation reactor.
4. The method of any one of embodiment 1 to 3 wherein a heater is provided to heat the gasesous stream and a booster compressor is provided downstream of the oxidation reactor and upstream of the heater.
5. A method of monitoring power recovery from an oxidation reaction producing a gaseous stream, the reaction being conducted in a continuous oxidation reactor fed with gaseous oxidant, comprising:
   (a) heating the gaseous stream to a temperature of at least 800°C;
   (b) feeding the gaseous stream to a turbine stage of a gas ICOCGT comprising a turbine coupled to a compressor, where the compressor compresses the gaseous oxidant fed to the reactor;
   (c) monitoring the pressure at the turbine stage of the ICOCGT;
   (d) maintaining the pressure within the turbine stage of the ICOCGT within a pressure range above a minimum value corresponding to the power demand of the compressor to compress the gaseous oxidant feed to an oxidation reactor and below a maximum value set by the power or pressure limits of the gas turbine by adding gas to the gaseous stream.
6. The method of any one of embodiment 2 to 5 wherein the mass flow of the gas added to the gaseous stream is in the range of from about 0% to about 25% of the mass flow of the gaseous stream prior to addition of the gas.
7. The method of any one of embodiment 2 to 6 wherein the mass flow of the gas added to the gaseous stream is in the range of from about 6% to about 15% of the mass flow of the gaseous stream prior to addition of the gas.
8. The method of any one of embodiment 2 to 7 wherein the gas is steam or air.
9. The method of any one of embodiments 1 to 8 comprising heating the gaseous stream to a temperature in the range of from 800°C to about 1350°C.
10. The method of embodiment 9 comprising heating the gaseous stream to a temperature in the range of from 800°C to about 1100°C.
11. The method of any one of embodiments 1 to 8 comprising heating the gaseous stream to a temperature of about 1050°C.
12. The method of any one of embodiments 1 to 11 wherein the gaseous stream is heated with a direct heater.
13. The method of any one of embodiments 1 to 11 wherein the gaseous stream is heated with an indirect heater.
14. The method of embodiment 12 wherein the gaseous stream is heated with a catalytic combustion unit prior to heating with a direct heater.
15. The method of embodiment 13 wherein the gaseous stream is heated with a catalytic combustion unit prior to heating with an indirect heater.
16. The method of any one of embodiments 1 to 15 wherein the gaseous stream is heated with an interchanger.
17. The method of any one of embodiments 1 to 16 wherein the gaseous stream is treated by a scrubber.
18. A continuous reactor for an oxidation reaction producing a gaseous stream, the reactor comprising:
   (a) a vessel comprising an oxidant inlet and a gaseous stream outlet; and
   (b) a power recovery system connected to the gaseous stream outlet comprising:
      (b1) a heater for heating the gaseous stream connected downstream of the gaseous stream outlet; and
      (b2) a gas turbine connected downstream of the heater comprising a turbine coupled to a compressor, where the compressor is connected to the oxidant inlet such that, when the reactor is in use, the compressor compresses the gaseous oxidant fed to the reactor.
19. The reactor of embodiment 18 wherein the power recovery system comprises a gas inlet for adding gas to the gaseous stream.
20. The reactor of embodiment 18 of embodiment 19 wherein the heater is a direct heater.
21. The method of embodiment 18 or embodiment 19 wherein the heater is an indirect heater.
22. The reactor of any one of embodiments 18 to 21 wherein a catalytic combustion unit is provided upstream of the heater.
23. The reactor of embodiment 22 wherein a scrubber is provided between the catalytic combustion unit and the heater.
24. The reactor of any one of embodiments 18 to 23 wherein the power recovery system comprises an expander or booster compressor provided downstream of the gas turbine compressor on the gaseous oxidant inlet to the oxidation reactor.
25. The reactor of any one of embodiments 18 to 24 wherein the power recovery system comprises a booster compressor downstream of the oxidation reactor and upstream of the heater.
26. A process of oxidising a precursor to an aromatic carboxylic acid or ester thereof in a liquid phase reaction mixture, whereby energy is recovered from the oxidation reaction, comprising:
   (a) contacting one or more precursors of the aromatic carboxylic acid with an oxidant, in the presence of a catalyst and a liquid phase solvent, in a continuous oxidation reactor fed with gaseous oxidant to produce aromatic carboxylic acid and a gaseous stream;
   (b) heating the gaseous stream to a temperature of at least 800°C;
   (c) feeding the gaseous stream to a gas turbine comprising a turbine coupled to a compressor, where the compressor compresses the gaseous oxidant fed to the reactor.
27. The process of embodiments 26 further comprising (d) generating steam from the gaseous stream after step (c).
28. The process of embodiment 26 further comprising (d) generating electricity from the gaseous stream after step (c).
29. The process of embodiment 26 further comprising (d) removing CO and NOx from the gaseous stream after step (c).
30. The method of embodiment 1 further comprising (c) generating steam from the gaseous stream after step (b).
31. The method of embodiment 1 further comprising (c) generating electricity from the gaseous stream after step (b).
32. The method of embodiment further comprising (c) removing CO and NOx from the gaseous stream after step (b).
33. The method of embodiment 5 further comprising (e) generating steam from the gaseous stream after step (b).
34. The method of embodiment 5 further comprising (e) generating electricity from the gaseous stream after step (b).
35. The method of embodiment 5 further comprising (e) removing CO and NOx from the gaseous stream after step (b).
36. The method of embodiment 5 further comprising (e) heating a feed to another reactor by direct heat exchange with the gaseous feed out of the gas turbine.
37. The method of embodiment 5 further comprising (e) heating a feed to another reactor by using the gaseous stream out of the gas turbine to raise high pressure steam.
38. The reactor of embodiment 18 wherein the power recovery system further comprises (b3) generating steam from the gaseous stream after step (b2).
39. The reactor of embodiment 18 wherein the power recovery system further comprises (b3) generating electricity from the gaseous stream after step (b2).
40. The reactor of embodiment 18 wherein the power recovery system further comprises (b3) removing CO and NOx from the gaseous stream after step (b2).

## Claims

1. A method for recovering power from an oxidation reaction producing a gaseous stream, the reaction being conducted in a continuous oxidation reactor fed with gaseous oxidant, comprising:
(a) heating the gaseous stream to a temperature of at least 800°C;
(b) feeding the gaseous stream to a turbine stage of a gas turbine comprising a turbine coupled to a compressor, where the compressor compresses the gaseous oxidant fed to the reactor.

2. The method of claim 1 wherein prior to, or simultaneously with, feeding the gaseous stream to the turbine, gas is added to the gaseous stream.

3. The method of claim 1 or claim 2 wherein an expander or booster compressor is provided downstream of the gas turbine compressor on the gaseous oxidant inlet to the oxidation reactor.

4. The method of any one of claims 1 to 3 wherein a heater is provided to heat the gaseous stream and a booster compressor is provided downstream of the oxidation reactor and upstream of the heater.

5. The method of any one of claims 2 to 4 wherein the mass flow of the gas added to the gaseous stream is in the range of from about 0% to about 25% of the mass flow of the gaseous stream prior to addition of the gas, optionally wherein the mass flow of the gas added to the gaseous stream is in the range of from about 6% to about 15% of the mass flow of the gaseous stream prior to addition of the gas.

6. The method of any one of claims 2 to 5 wherein the gas is steam or air.

7. The method of any one of claims 1 to 6 comprising heating the gaseous stream to a temperature in the range of from 800°C to about 1350°C, optionally comprising heating the gaseous stream to a temperature in the range of from 800°C to about 1100°C, optionally comprising heating the gaseous stream to a temperature of about 1050°C.

8. The method of any one of claims 1 to 7 wherein the gaseous stream is heated with a direct heater, optionally wherein the gaseous stream is heated with an indirect heater.

9. The method of claim 8 wherein the gaseous stream is heated with a catalytic combustion unit prior to heating with a direct heater, optionally wherein the gaseous stream is heated with a catalytic combustion unit prior to heating with an indirect heater.

10. The method of any one of claims 1 to 9 wherein the gaseous stream is heated with an interchanger.

11. The method of any one of claims 1 to 10 wherein the gaseous stream is treated by a scrubber.

12. A continuous reactor for an oxidation reaction producing a gaseous stream, the reactor comprising:
(a) a vessel comprising an oxidant inlet and a gaseous stream outlet; and
(b) a power recovery system connected to the gaseous stream outlet comprising:
(b1) a heater for heating the gaseous stream connected downstream of the gaseous stream outlet; and
(b2) a gas turbine connected downstream of the heater comprising a turbine coupled to a compressor, where the compressor is connected to the oxidant inlet such that, when the reactor is in use, the compressor compresses the gaseous oxidant fed to the reactor.

13. The reactor of claim 12 wherein the power recovery system comprises a gas inlet for adding gas to the gaseous stream.

14. The reactor of claim 12 or claim 13 wherein the heater is a direct heater, optionally wherein the heater is an indirect heater.

15. The reactor of any one of claims 12 to 14 wherein a catalytic combustion unit is provided upstream of the heater.

16. The reactor of claim 15 wherein a scrubber is provided between the catalytic combustion unit and the heater.

17. The reactor of any one of claims 12 to 16 wherein the power recovery system comprises an expander or booster compressor provided downstream of the gas turbine compressor on the gaseous oxidant inlet to the oxidation reactor.

18. The reactor of any one of claims 12 to 17 wherein the power recovery system comprises a booster compressor downstream of the oxidation reactor and upstream of the heater.

19. The method of claim 1 further comprising (c) generating steam from the gaseous stream after step (b), optionally further comprising (c) generating electricity from the gaseous stream after step (b), optionally further comprising (c) removing CO and NOx from the gaseous stream after step (b).

20. The reactor of claim 12 wherein the power recovery system further comprises (b3) generating steam from the gaseous stream after step (b2), optionally, wherein the power recovery system further comprises (b3) generating electricity from the gaseous stream after step (b2), optionally wherein the power recovery system further comprises (b3) removing CO and NOx from the gaseous stream after step (b2).
